# EUROPÄISCHE PATENTSCHRIFT

(11) **EP 2 406 626 B1**
(45) Veröffentlichungstag und Bekanntmachung des Hinweises auf die Patenterteilung: **16.01.2013**
(21) Anmeldenummer: 10707908.9
(22) Anmeldetag: 10.03.2010
(51) Int. Cl.: G01N 33/50, G01N 33/84

(54) **VERFAHREN UND MITTEL ZUM NACHWEIS DER AKTIVITÄT VON OSTEOKLASTEN**
METHOD AND MEANS FOR DETECTING THE ACTIVITY OF OSTEOCLASTS
PROCÉDÉ ET MOYENS DE DÉTECTION DE L'ACTIVITÉ D'OSTÉOCLASTES

(30) Priorität: 13.03.2009 DE 102009013957
(43) Veröffentlichungstag der Anmeldung: 18.01.2012
(73) Patentinhaber: Technische Universität Dresden, 01069 Dresden (DE)
(72) Erfinder: DIETER, Peter, 01157 Dresden (DE); LUTTER, Anne-Helen, 04356 Leipzig (DE); HEMPEL, Ute, 01307 Dresden (DE)
(74) Vertreter: Kailuweit & Uhlemann Patentanwälte
(86) Internationale Anmeldenummer: PCT/EP2010/053054
(87) Internationale Veröffentlichungsnummer: WO 2010/103053

(56) Entgegenhaltungen:
- WO-A1-94/26872
- WO-A1-03/089022
- WO-A1-2008/128342
- DE-A1-102004 021 229
- US-A- 5 861 306
- JONES S J ET AL: "SIMULATION OF BONE RESORPTION-REPAIR COUPLING IN VITRO" ANATOMY AND EMBRYOLOGY, SPRINGER INTERNATIONAL, BERLIN, DE LNKD- DOI:10.1007/BF00187292, Bd. 190, Nr. 4, 1. April 1994 (1994-04-01) , Seiten 339-349, XP008038542 ISSN: 0340-2061
- BERNHARDT A ET AL: "In vitro osteogenic potential of human bone marrow stromal cells cultivated in porous schaffolds from mineralized collagen" JOURNAL OF BIOMEDICAL MATERIALS RESEARCH PART A, Bd. 90A, Nr. 3, 9. Juli 2008 (2008-07-09), Seiten 852-862, XP002583805 DOI: 10.1002/jbm.a.32144
- LUTTER AH ET AL: "A Novel Resporption Assay for Osteoclast Functionality Based on an Osteoblast-Derived Native Extracellular Matrix" JOURNAL OF CELLULAR BIOCHEMISTRY, Bd. 109, 27. Januar 2010 (2010-01-27), Seiten 1025-1032, XP002583806 DOI: 10.1002/jcb.22485

## Beschreibung

Die Erfindung betrifft ein Verfahren und Mittel zum Nachweis der Resorptionsaktivität von Osteoklasten, insbesondere zur Anwendung in der Medizin, sowie in der biowissenschaftlichen und pharmazeutischen Forschung.

Knochen ist ein dynamisches Gewebe, welches einem ständigen Umbau (Knochenabbau und Knochenaufbau) unterliegt. Knochenaufbauende Zellen (Osteoblasten) und Knochenabbauende Zellen (Osteoklasten) stehen in gesundem Knochen in einem Gleichgewicht, das sich im Alter zu den Osteoklasten hin verschiebt. Viele Knochenkrankheiten gehen auf Fehlfunktionen in Osteoklasten oder Störungen im Gleichgewicht zurück und werden intensiv untersucht. Bei der Osteoporose ist z. B. die Resorptionsaktivität der Osteoklasten gestört.

DE 10 2004 021 229 A1 offenbart ein Verfahren zur Herstellung bioaktiver Osteoblasten stimulierender Oberflächen durch Modifikation mit amorphem Siliciumdioxid (Silica) und/oder Siliconen, sowie mittels enzymatischer Modifikation mit einem Polypeptid, welches eine Silicatein-α- oder Silicatein-β-Domaine umfasst. Auf einer derart modifzierten Oberfläche wird *in vitro* eine mineralisierten Calciumphosphat-haltigen Matrix durch die Osteoblastenlinie SAOS-2 in Gegenwart von β-Glycerophosphat synthetisiert. Mit der speziellen Silica-modfizierten Oberfläche, die nochmals mit Kollagen beschichtet wird, wird eine bessere Mineralisierung erreicht, als mit einer allein mit Kollagen beschichten Matrix. Jedoch ist in jedem Fall die erreichte Mineralisierung unregelmäßig und führt nicht zu einer vollständigen Bedeckung der Oberfläche.

Bisherige Verfahren zur Messung der Resorptionsaktivität von Osteoklasten *in vitro* sind schwierig zu quantifizieren, sind teilweise unflexibel in der Anwendung mit verschiedenen Spenderorganismen und erfordern spezielle Messgeräte zur Datenerfassung.

Auf dem Markt sind folgende Testkits:
1. Der Osteoclast Culture Kit der Kamiya Biomedical Company (Seattle WA, USA) arbeitet auf der Basis von kompakten Dentin-Scheiben in Verbindung mit speziellen Ratten-Vorläuferzellen V-1, die zu Osteoklasten differenzieren. Dentin ist knochenähnlich und besteht zu ca. 70 % aus Calciumhydroxylapatit (hauptsächlich Phosphat und Calcium) und zu 20 % aus organischen Bestandteilen (davon sind 90% Kollagen). Die restlichen 10 % sind Wasser. Die durch die Osteoklastenaktivität entstandenen Resorptionslakunen sind nachteilig nur nach Hematoxylin-Färbung oder mit dem Elektronenmikroskop zu quantifizieren. Außerdem sind die Dentin-Scheiben undurchlässig für Licht und damit nachteilig Fluoreszenz- und Lichtmikroskopisch nicht auswertbar.
2. Die OAAS^{™}-Plates der OCT USA, Inc. (Buena Park, CA, USA) sind mit synthetisch hergestellten karboniertem Calciumphosphat beschichtete Zellkulturplatten. Eine Quantifizierung der Osteoklastenaktivität ist nur bedingt möglich, da eine Erkennung von Resorptionslakunen im Verhältnis zum Hintergrund schwierig ist. Eine Auswertung ist zwar über eine CCD-Kamera möglich, aber der Kontrast zwischen Fressfläche und Matrix ist schlecht, da die Platten braun färben und nicht schwarz.
3. Der BD Biocoat^{™} Osteologic^{™} Discs der BD Biosciences (Bedford, MA, USA) beruht ebenfalls auf mit synthetisch hergestelltem Calciumphosphat beschichteten Platten, welche aber in diesem Fall mit der von Kossa-Methode gefärbt werden, bevor eine Quantifizierung erfolgt. Das Problem dieses Test liegt in dem künstlichen Substrat, was den Zellen angeboten wird und welches das Resorptionsverhalten der Zellen beeinflussen kann. In der WO 2008/153814 A1 werden die BD Biocoat^{™} Osteologic^{™} Discs beispielsweise angewendet.
4. Gegenstand der US20080299604A1 ist der unter dem Namen OsteoLyse^{™} Assay Kit von der Lonza Walkersville, Inc. vertriebene Kit. Dieser beruht auf der quantitativen Bestimmung von Kollagen-Abbauprodukten. Verwendet werden mit Europium-markiertem Kollagen beschichtete Platten. Gemessen wird der Kollagenabbau und die durch Freisetzung des Europiums hervorgerufene Fluoreszenz. Das System funktioniert nur mit speziellen auf das Testsystem abgestimmten humanen Osteoklast-Vorläuferzellen, was keine Variabilität ermöglicht.
5. Der OsteoAssay^{™} Human Bone Plate der Lonza Walkersville, Inc. (Walkersville MD, USA) beruht auf einer dünnen Schicht aus adherenten humanen Knochenpartikeln, die eine Matrix für primäre humane oder nicht-humane Osteoklasten bildet. Gemessen wird auch hier der Kollagenabbau im Überstand. Eine direkte Korrelation mit der Resorptionsfläche bzw. mit der Anzahl der Resorptionslakunen ist in diesem Test nicht möglich.
6. Der CalciFluor™ Assay der Lonza Walkersville, Inc. beruht auf der Messung von freiem Calcium, welches während der Resorption freigesetzt wird. Problematisch ist dabei, dass spezielles Medium verwendet werden muss, um zu verhindern, dass Calcium, welches in normalem Medium vorhanden ist, die Messwerte verfälscht.

US5849569 offenbart ein Substrat für die Kultur von Knochenzellen *in vitro* zur Bestimmung deren Aktivität. Dieses Substrat besteht aus synthetisch hergestelltem Calciumphosphat, welches durch eine Beschichtung im Solgel-Verfahren erhalten wird.

W02007135531A2 offenbart ein Bildbearbeitungssystem für die Auswertung von Bildern aus Osteoklastenaktivitätsassays.

WO 94/26872 A1 offenbart Verfahren und Mittel zur Bestimmung der Resorptionsaktivität von Osteoklasten. Dabei werden die Osteoklasten mit einem synthetischen Calciumphosphatfilm versetzt, der bevorzugt Calciumhydroxylapatit und Tricalciumphosphat enthält. Das nicht-resorbierte Calciumphosphat wird anschließend quantifiziert.

Aufgabe der Erfindung ist es, ein vereinfachtes und verbessertes Verfahren und Mittel für dessen Durchführung anzugeben, um die Resorptionsaktivität von Osteoklasten *in vitro* nachzuweisen und zu quantifizieren. Das Verfahren soll auf Osteoklasten, die aus unterschiedlichen Zelllinien hervorgehen, und native Zellen unterschiedlicher Spezies anwendbar sein.

Die vorliegende Erfindung entstand dabei in dem Bestreben einen Spezies-unabhängigen, einfach quantifizierbaren und physiologischen Osteoklasten-Resorptionsassay zu entwickeln.

Die Aufgabe wird erfindungsgemäß gelöst durch ein Verfahren zur Bestimmung der Resorptionsaktivität von Osteoklasten mit den Schritten:
a.) Inkubation von Osteoklasten mit einer biomineralisierten, calciumphosphathaltigen Matrix, die durch Osteoblasten *in vitro* gebildet wurde,
b.) Quantifizierung des nicht resorbierten Calciumphosphats.

Unter Resorptionsaktivität von Osteoklasten wird die knochenabbauende Aktivität der Osteoklasten verstanden.

Durch die Osteoblasten wird vorteilhaft *in vitro* eine native biomineralisierte Knochenmatrix auf einem Trägermaterial aufgebaut, d. h. eine Matrix, welche in ihrer Zusammensetzung und in ihrer Struktur nahezu dem natürlichen Knochen entspricht.

Die erfindungsgemäß verwendete Matrix, die durch Biomineralisation durch Osteoblasten *in vitro* erhalten wurde, unterscheidet sich von einer Beschichtung aus synthetisch hergestelltem Calciumphosphat insbesondere in folgenden Punkten:
1. Die Matrix enthält (wie der natürliche Knochen) circa 10 bis 30 %, bevorzugt 15 bis 25 % organische Materialien (z. B. Kollagen Typ I, Osteonectin, Osteocalcin (ggf. in geringeren Mengen), Osteopontin, Proteoglycane, Bone Sialoprotein) und circa 70 bis 85 % aus anorganischen Stoffen (vor allem Calciumphosphat in Form von Hydroxylapatit (Ca₁₀[PO₄]₆[OH]2)).
2. Die Struktur der Matrix ähnelt dem natürlichen Knochen, d. h. sie besteht aus einem Geflecht aus Strukturproteinen (wie Kollagen Typ I, Osteopontin) an die über Calciumbindende Proteine (wie Calciumnectin) Calciumphosphat (insbesondere in der Form von Hydroxylapatit) gebunden ist.

Da die erfindungsgemäß verwendete Matrix in ihrer Struktur und ihrer Zusammensetzung dem natürlichen Knochen entspricht, ermöglicht die Erfindung vorteilhaft die Resorptionsaktivität von Osteoklasten unter nahezu physiologischen Bedingungen zu testen.

Die erfindungsgemäß verwendete Matrix besteht vorzugsweise (abgesehen von Resten des Zellkulturmediums) allein aus biologisch hergestellten Komponenten, d. h. enthält kein synthetisch hergestelltes Calciumphosphat. Auch der Einsatz von Crosslinkern oder anderen Materialien zur Fixierung der Matrix auf dem Trägermaterial ist nicht notwendig.

Die erfindungsgemäß verwendete Matrix kann vorteilhaft auf gängigen Zellkulturschalen (z. B. aus Polystyren oder Polycarbonat), keramischen oder metallischen Oberflächen (wie Titan), Glas oder anderen bekannten, bevorzugt durchsichtigen Trägermaterialien durch Inkubation mit Osteoblasten *in vitro* erhalten werden. Vorteilhaft ist, dass keine vorherige Beschichtung (kein pre-coating) der Oberflächen notwendig ist, d. h. die Zellen werden im Zellkulturmedium direkt auf den genannten Oberflächen inkubiert. Die erfindungsgemäß verwendete Matrix wird somit direkt auf den genannten Oberflächen ausgebildet. Die Größe der Schalen (z. B. 6-well, 24-well, 48-well, 96-well, Petrischalen aller bekannter Größen) kann beliebig gewählt werden.

Der Begriff Osteoblasten umfasst dabei Osteoblastenzelllinien, einschließlich Osteosarcoma Zelllinien mit Osteoblasteneigenschaften (wie z. B: SAOS-2-Zellen) und native Osteoblasten.

Die Dicke der erfindungsgemäß verwendeten Matrix kann vorteilhaft durch die Zeitdauer der Inkubation mit Osteoblasten eingestellt werden. Bevorzugt ist die Schicht jedoch dünn genug um eine lichtmikroskopische oder eine einfache densitometrische oder photometrische Auswertung zu erlauben. Auch die Auswertung mit einer CCD-Kamera, digitalen Photokamera oder einem Desktop Scanner ist möglich. Vorteilhaft ist die Homogenität der erfindungsgemäß verwendeten Matrix, d. h. die Schicht enthält keine Lücken. Die Matrix ist durchgängig mindestens 100 nm dick. Bevorzugte Schichtdicken liegen im Bereich von 0,4 - 0,8 µm bezogen auf Hydroxylapatit.

Die Herstellung der Matrix erfolgt bevorzugt durch Inkubation einer Osteoblastenzelllinie, bevorzugt von SAOS-2 Zellen, auf dem Trägermaterial in einem Medium.

Die Inkubation der Zellen erfolgt bevorzugt in einem Standardzellkulturmedium (welches Salze, Aminosäuren, Vitamine Glucose, Desoxyribonucleoside und Ribonucleoside und Puffersubstanzen, wie z. B. Alpha MEM) und Serum (bevorzugt 5 % bis 20 % Serum, vorzugsweise Foetales Kalbserum) enthält. Das Medium wird mit Ascorbat (bevorzugt 100 bis 500 µmol/l) und Phosphat, insbesondere organisch gebundenem Phosphat, bevorzugt β-Glycerolphosphat (bevorzugt 2 bis 20 mmol/1) angereichert. Die Endkonzentration an Ascorbat beträgt demnach bevorzugt mindestens 100 µmol/l. Standardzellkulturmedium (wie Alpha-MEM) enthalten häufig 300 µmol Ascorbat/l (50 mg/1). Die Endkonzentration an Ascorbat beträgt demnach besonders bevorzugt 400 bis 800 µmol/l. Die bevorzugte Endkonzentration an β-Glycerolphosphat oder anderem organisch gebundenen Phosphat beträgt 2 bis 20 mmol/l. Die Konzentration an Calciumsalzen (z. B. CaCl₂ x HO) beträgt bevorzugt 0,1 bis 0,5 g/l besonders bevorzugt 0,1 bis 0,3g/l.

Die Inkubation zur Bildung der Matrix erfolgt bevorzugt für 15 bis 35 Tage, besonders bevorzugt 20 bis 30 Tage. Das Medium wird bevorzugt täglich bis alle 5 Tage, besonders bevorzugt alle 3 bis 4 Tage gewechselt, um eine optimale Differenzierung und eine ausreichende Dichte der Calciumphosphat Matrix zu gewährleisten. Die CO₂-Konzentration liegt bevorzugt im Bereich von 4-12 %, besonders bevorzugt bei 5-7 % und die O₂-Konzentration liegt bevorzugt im Bereich von 10-30 %, besonders bevorzugt bei 17 bis 23 % oder 20±2 % (physiologische Konzentration).

Für das Aufbringen der Matrix ist keine maschinelle Unterstützung notwendig. Jedoch kann die Herstellung durch den Einsatz von Pipettierrobotern automatisiert werden.

Eine gleichmäßige Beschichtung lässt sich über die Bestimmung des Calcium-PhosphatGehaltes kontrollieren.

Nach erfolgter Matrixproduktion werden die noch vorhandenen Osteoblasten (bzw. Osteocyten) von der Matrix entfernt. Dies geschieht bevorzugt mit wässriger Ammoniaklösung oder einer Harnstofflösung. Anschließend werden die Platten mit Wasser oder einem geeigneten Puffer (wie z. B. einem Phosphatpuffer) gewaschen

Auf die so hergestellte Matrix können die Osteoklasten unmittelbar ausgebracht werden.

Wird die Matrix nicht sofort verwendet, kann diese vorteilhaft getrocknet und bei Raumtemperatur gelagert werden. Auch die Lagerung (z. B. in PBS) bei 4°C ist problemlos möglich, genauso wie das Einfrieren bei -20°C bis -80°C. Die Matrix kann auch mit üblichen Methoden (z. B. UV- oder Gammabestrahlung) sterilisiert werden. Dies ist jedoch nicht unbedingt notwendig wenn ausschließlich sterile Materialien eingesetzt wurden.

Zum Nachweis der Resorptionsaktivität der Osteoklasten (auch Fressaktivität genannt) werden diese auf der erfindungsgemäß verwendeten Matrix inkubiert, wobei die Inkubation in dem oben genannten Zellkulturmedium oder anderen bekannten Medien erfolgen kann. Der Test eignet sich auch für die Anwendung auf Osteoklastenvorläuferzellen (oder deren Zellinien), wobei diese durch Zugabe von geeigneten Zytokinen auf der erfindungsgemäßen Matrix in Osteoklasten differenziert werden.

Die Inkubation erfolgt bevorzugt für 3 bis 20 Tage, abhängig von dem jeweiligen Zelltyp. Während dieser Zeit bauen die Osteoklasten die Matrix und insbesondere das darin enthaltene Calciumphosphat ab.

Das verbleibende Calciumphosphat wird anschließend quantifiziert. Durch Vergleich mit dem Ausgangswert bzw. einer Kalibriergerade wird der Anteil an resorbiertem Calciumphosphats berechnet, bzw. die resorbierte Fläche im Verhältnis zur nicht resorbierten Fläche.

Der Test ist nach beliebigen Zeitpunkten abstoppbar. Dabei kann entweder ein Ablösen der Zellen erfolgen oder eine Fixierung der Zellen auf der Matrix für anschließende Färbungen. Bevorzugt erfolgt das Abstoppen durch Abtöten und Ablösen der Osteoklasten mit wässriger Ammoniaklösung oder einer Harnstofflösung. Die verbleibende Matrix wird anschließend mit Wasser oder einem geeignetem Puffer gewaschen.

Die Matrix muss für die Quantifizierung der Resorptionsaktivität nicht fixiert werden. Wenn Zellen auf der Matrix betrachtet werden sollen, dann können diese z. B. mit Paraformaldehyd (3,7 -10%), einem Aceton/Methanol-Gemisch oder mit anderen herkömmlichen Fixierungsmethoden fixiert und anschließend gefärbt werden.

Zur Quantifizierung des Calciumphosphats können unterschiedliche Methoden zur Anwendung kommen.
Bevorzugt wird eine Silberabscheidung (Austausch von Calcium gegen Silber) durch Zugabe von löslichen Silbersalzen in Gegenwart von Reduktionsmitteln verwendet. Bei dieser Methode, von Kossa-Färbung genannt, ist eine deutliche Unterscheidung (schon visuell) zwischen nativer Matrix (schwarz) und resorbierten Löchern (durchsichtig) möglich. Die Quantifizierung kann dann über verschiedene Wege erfolgen. Die einfachste Möglichkeit erfordert kein spezielles Gerät zum Auslesen, sondern besteht darin die verbleibende Matrix bzw. das Trägermaterial einzuscannen und danach die hellen Bereiche über eine Software wie z. B. Image Quant oder ImageJ zu analysieren. Vorteil hierbei ist, dass ein ganzes Well (Kavität) analysiert werden kann und nicht nur ein Teilbereich, womit eine ungleichmäßige Verteilung von (durch die Osteoklastenaktivität entstandenen) Löchern im Well keinen Einfluss auf die Ergebnisse hat. Die Reproduzierbarkeit und Aussagekraft dieser integrativen Analyse des ganzen Well und damit der gesamten Zellpopulation wird durch die erfindungsgemäße homogene Beschichtung erheblich verbessert.

Zusätzlich zu der Quantifizierung der Osteoklastenaktivität kann ein Nachweis von zellulären Markern, bevorzugt durch Immunofluoreszenzfärbung oder Immunohistochemie. Vorteilhaft stört die erfindungsgemäße Matrix einen derartige Färbung und mikroskopischen Nachweis (z. B. mit einem klassischen Fluoreszenzmikroskop oder einem Konfokalmikroskop) nicht.

Dieser Nachweis erfolgt bevorzugt vor der Durchführung der von Kossa-Färbung. Vorteilhaft ist, dass die für die Immunofluoreszenzfärbung oder Immunohistochemie verwendete Reagenzien, die z. T. auf den Zellen verbleiben (Antikörper, Fixierungsmittel, wie Formalin, Blockierungsmittel, wie Rinderserumalbumin, Flourezenzfarbstoffe) die anschließende Quantifizierung des Calciumphosphats (bevorzugt mit der von Kossa-Färbung) nicht stören. Alternativ kann die Quantifizierung des Calciumphosphats durch andere bekannte Nachweismethoden für Calcium (z. B. mit Calcon und/oder Calcein oder Arsenazo-III oder als Hexacyanoferrat) oder Phosphat oder auch radioaktive Markierung des Calciums oder Phosphors erfolgen.

Das erfindungsgemäße Verfahren funktioniert vorteilhaft mit Osteoklasten von verschiedenen Organismen und Zelltypen, insbesondere primären Mausosteoklastenzellen (primary mouse bone marrow monocytes), Maus-Osteoklasten-Zelllinien (wie z. B. RAW 264.7), primären Rattenosteoklastenzellen, Ratten-Osteoklasten-Zelllinien, Osteoklasten, die aus primären humanen peripheren mononuklären Blutzellen (PBMC - Peripheral Blood Mononuclear Cells) oder primären humanen CDI4⁺-Zellen durch Differenzierung erhalten wurden, primäre humane Zellen aus dem Knochenmark (human bone marrow cells), OsteoklastenVorläuferzellen von Lonza, gleichermaßen.

Schon während der Kultur kann man lichtmikroskopisch (bevorzugt mit Kontrast-Mikroskopie) die Entstehung von Löchern verfolgen. Auch unterschiedliche Stadien der Differenzierung der Osteoklasten können lichtmiki-oskopisch, insbesondere mittels Fluoreszenzfärbungen verfolgt werden.

Gegenstand der Erfindung ist auch die Verwendung eines Kits zur Bestimmung der Resorptionsaktivität von Osteoklasten, wobei der Kit die folgenden Bestandteile enthält:
a.) eine Matrix, die durch Abscheidung von Calciumphosphat durch Osteoblasten *in vitro* erhalten wurde,
   sowie gegebenenfalls mindestens einen der weiteren Bestandteile:
b.) Zellkulturmedium, Differenzierungsfaktoren (wie z. B. RANKL)
c.) Fixierungslösung, Waschpuffer,
d.) Sibernitratlösung, Reduktionsmittel
e.) eine Kalibriergerade,
f.) eine oder mehrere Osteoklastenzellinien,
g.) eine Bedienungsanleitung.
h.) Programmhinweise und eventuell Hinweise bzgl. Auswahl und Benutzung eines Scanners.

Vorteilhaft kann der Anwender Osteoklastenzellen seiner Wahl verwenden. Sofern Osteoklastenzelllinien dem Kit begelegt werden, dienen diese bevorzugt als Vergleichsproben bzw. positive Kontrollen.

Die Erfindung betrifft auch die Verwendung des erfindungsgemäßen Verfahrens, der Matrix und des Kits zur Bestimmung der Resorptionsaktivität von Osteoklasten, zur Anwendung in der biologischen oder medizinischen Forschung (z. B. zum Studium der Osteoklastendifferenzierung), der pharmakologischen Forschung (z. B. zur Untersuchung und Entwicklung von Stoffen, welche die Osteoklastenaktivität- und/oder differenzierung beeinflussen) und zur Anwendung in der Medizin (z. B. zur Diagnose der Osteoklastenaktivität- und/oder differenzierung). Ausserdem besteht durch die Erfindung die Möglichkeit Cokulturen von Osteoblasten und Osteoklasten unter physiologischen Bedingungen zu untersuchen.

### Ausführungsbeispiele:

Die Erfindung wird nachfolgend durch Abbildungen und Ausführungsbeispiele näher erläutert, ohne die Erfindung auf diese zu beschränken:

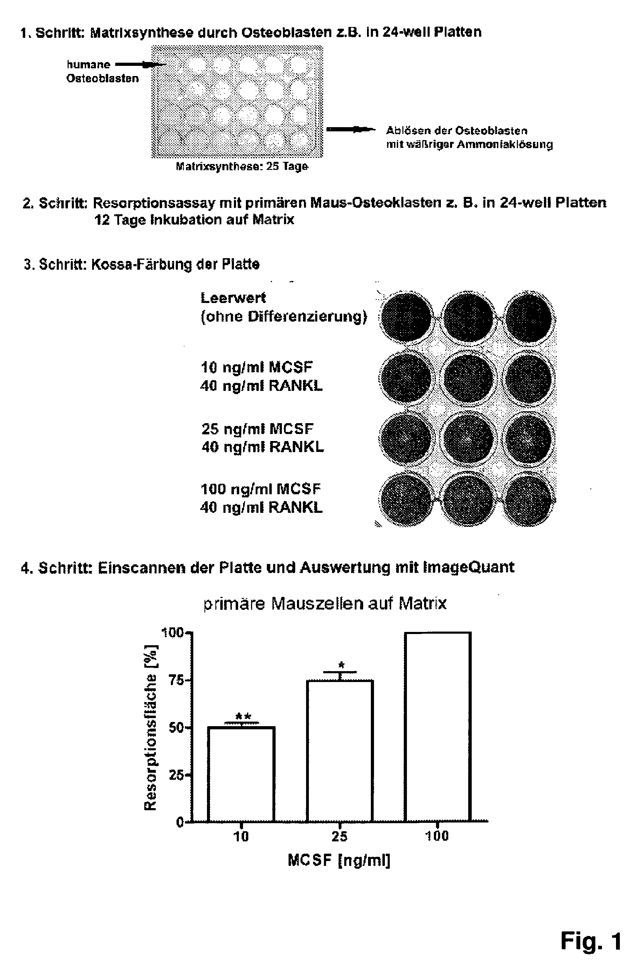

**Fig. 1** zeigt schematisch den Ablauf des erfindungsgemäßen Verfahrens, einschließlich des Schritts der Matrixherstellung. Der Ablauf gliedert sich in zwei Phasen. In Phase I findet die Matrixsynthese mit Osteoblasten statt, welche nach Produktion der Matrix (ca. 25 Tage) von der Matrix entfernt werden. Die anschließende Phase II beinhaltet die Verwendung der Matrix für den Osteoklasten-Resorptionsassay. Dabei wurde die Methode in diesem Beispiel angewandt um die Resorptionsaktivität von primären Mausosteoklastenvorläuferzellen (primary mouse bone marrow monocytes) in Abhängigkeit der Konzentration an MCSF (Makrophagen Koloniestimulierender Faktor) zu untersuchen. Die Resorptionsaktivität der differenzierten Osteoklasten ist (wie bereits zuvor gezeigt wurde - Lees RL et al. 1999 J Bone Miner Res. 14(6), 937-45) abhängig von der Menge an zugegebenen MCSF, wenn der Differenzierungsfaktor RANKL ("Receptor Activator of NF-κB Ligand") (40 ng/ml) vorhanden ist. Das Diagramm in Fig. 1 zeigt das die Untersuchung der MCSF-Abhängigkeit mit diesem Test signifikant nachweisbar ist.

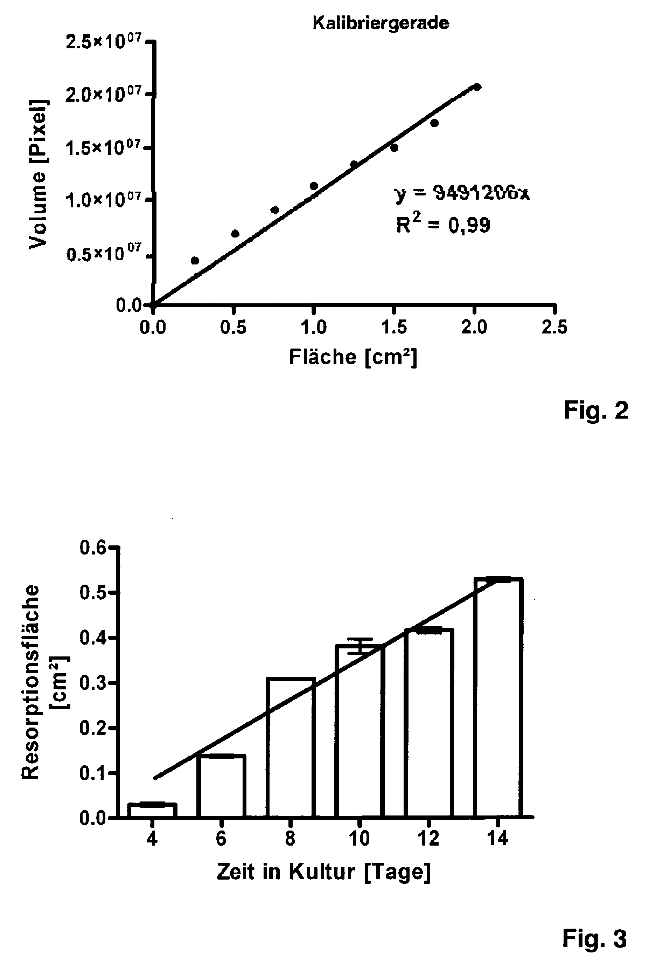

**Fig. 2** zeigt die Kalibriergerade zur Berechnung der Resorptionsfläche. Die Kalibriergerade wird durch partiellen Auftrag der Matrix erstellt.

Ein weiteres Beispiel für die Anwendung des Testsystem zeigt Fig. 3. Hier wurde die Zeitabhängigkeit der Resorptionsaktivität von einer durch RANKL- Zugabe (40 ng/ml) differenzierten Mausosteoklastenzelllinie (RAW 264.7) untersucht, dass heißt die Zunahme an Resorptionsfläche pro Tag kann mit diesem Test einfach und effizient verfolgt werden.

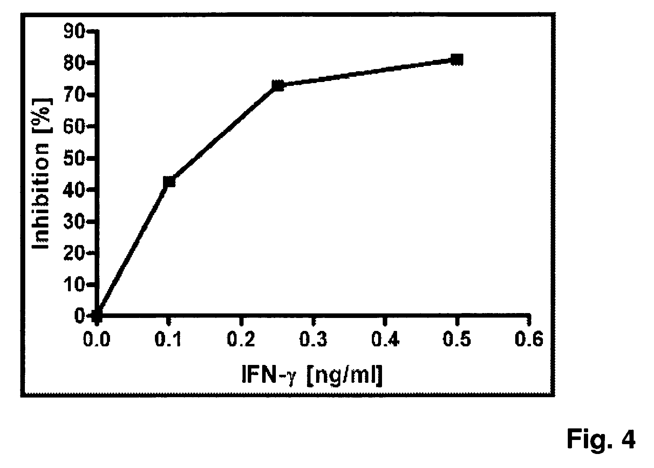

Des Weiteren ist es gerade in der medizinischen Forschung von besonderem Interesse Hemmstoffe bzw. Aktivatoren der Osteoklastenaktivität zu untersuchen. Beispielhaft wird in **Fig. 4** gezeigt, dass Osteoklastenaktivität von dem Inhibitor Interferon-y abhängig ist. Die Osteoklasten wurden durch RANKL-Zugabe (40 ng/ml) aus einer Mausosteoklastenvorläuferzelllinie (RAW 264.7) differenziert. Dabei wird eine Konzentrationsabhängige Hemmung gezeigt, die auch schon in der Literatur beschrieben wurde (Kamolmatyakul S, Chen W, Li YP. 2001, J. Dent. Res. 80 (1), 351-355). Damit kann man die Erfindung auch für Hemmstudien im Bezug auf die Osteoklasten-Resorptionsaktivität nutzen.

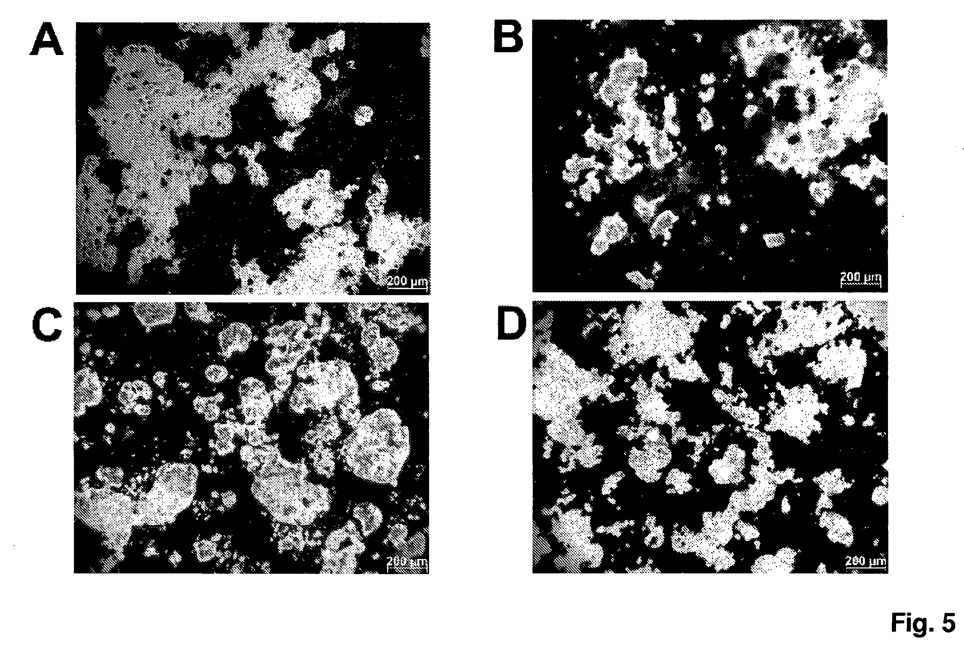

Ein wichtiger Vorteil des entwickelten Resorptionsassays besteht darin, dass Zelltypen von verschiedenen Organismen eingesetzt werden können. **Fig. 5** zeigt die resorbierte Matrix von differenzierten primären humanen CD14⁺ PBMC (A), primären humanen Osteoklasten Vorläuferzellen von Lonza GmbH (B), RAW 264.7 Zellen (C) und primären Mauszellen aus dem Knochenmark (D). Die Abbildungen zeigen, dass die verschiedenen Zelltypen gleichermaßen in der Lage sind, die Matrix abzubauen.

### Ausführungsbeispiel 1: Synthese einer knochenähnlichen physiologischen Matrix

Zur Synthese einer knochenähnlichen physiologischen Matrix wird in diesem Beispiel die humane Osteoblastenvorläuferzellinie SAOS-2 (DSMZ ACC 243, DSMZ, Braunschweig) verwendet. Die Zellen werden auf Zellkulturplatten ausgesät. Die Differenzierung der Zellen erfolgt durch Zugabe von Ascorbat (300 µmol/l) und β-Glycerophosphat (10 mmol/1) zum Medium (Alpha MEM, Biochrom, 10% FKS). Daraus ergeben sich folgende Endkonzentrationen: 600 µmol/l Ascorbat und 10 mmol/l β-Glycerophosphat. Inkubiert wird bei 5 % CO₂ und 20 % O₂ und 37°C. Das Medium wird alle 3 bis 4 Tage gewechselt, um eine optimale Differenzierung und eine ausreichende Dichte der Calciumphosphat Matrix zu gewährleisten. Nach 25 Tagen werden noch vorhandene Zellen mit Ammoniaklösung (20 mmol/1) von der Matrix entfernt. Die fertigen Platten werden anschließend gründlich mit PBS gewaschen und bei 4°C in PBS bis zur Verwendung gelagert.

### Ausfuhrungsbeispiel 2: Resorptionsphase durch Osteoklasten

Um das Resorptionsverhalten von aktiven Osteoklasten zu studieren, werden verschiedene Osteoklastenvorläuferzellen (s. Tabelle 1) auf die vorher gemäß Ausführungsbeispiel 1 hergestellten Matrixplatten ausgesät. Die Differenzierung der Zellen erfolgt in Kulturmedium (Alpha MEM, 20% Fötales Kälberserum - FKS) durch Zugabe von unterschiedlichen Zytokinen je nach Zelltyp (s. Tabelle 1). Das Medium wird alle 3 Tage gewechselt, um eine optimale Differenzierung zu gewährleisten. Unabhängig von den Zellen, wird die Resorptionsphase nach bestimmten Zeitpunkten beendet. Um eine optimale Auswertung der Platten zu ermöglichen, werden noch vorhandene Zellen mit Ammoniaklösung (20 mmol/1) von der Matrix entfernt. Die resorbierten Platten werden anschließend gründlich mit PBS gewaschen und bei 4°C in PBS bis zur Auswertung gelagert.

**TABELLE 1: OSTEOKLASTEN VORLÄUFERZELLEN**

| | **Spezies** | **Medium** | |
|---|---|---|---|
| Primäre Knochenmarkszellen | murin | Alpha MEM | |
| | | Hitzeinaktivierte FKS | 20 % |
| | | Penicillin/Streptavidin | 10000 E |
| | | L-alanyl-L-glutamin | 5 mmol/l |
| | | Murines RANKL | 40 ng/ml |
| | | Murines MCSF | 10 - 100 ng/ml |
| Primäre Knochenmarkszellen | human | Alpha MEM | |
| | | Hitzeinaktivierte FKS | 20 % |
| | | Penicillin/Streptavidin | 10000 E |
| | | L-alanyl-L-glutamin | 5 mmol/l |
| | | humanes RANKL | 10 - 100 ng/ml |
| | | humanes MCSF | 10 - 100 ng/ml |
| Primäre CD14⁺ PBMC | human | Alpha MEM | |
| | | Hitzeinaktivierte FKS | 20 % |
| | | Penicillin/Streptavidin | 10000 E |
| | | L-alanyl-L-glutamin | 5 mmol/l |
| | | humanes RANKL | 10 - 100 ng/ml |
| | | humanes MCSF | 10 - 100 ng/ml |
| Osteoclast precursor | human | Osteoclast differentiation medium (Lonza) | |
| cells (Poietics Osteoclast Precursor Cell System, Lonza, Wuppertal, DE) | | FKS | 10 % |
| | | Penicillin/Streptavidin | 10000 E |
| | | L-alanyl-L-glutamin | 5 mol/l |
| | | humanes RANKL | 10 - 100 ng/ml (von Firma empfohlen 66 ng/ml) |
| | | humanes MCSF | 10 - 100 ng/ml (von Firma empfohlen 33 ng/ml) |
| RAW 264.7 (ATCC number: TIB71^{™}, LGC Standards GmbH, Wesel, DE) | murin | Alpha MEM | |
| | | Hitzeinaktivierte FKS | 20 % |
| | | Penicillin/Streptavidin | 10000 E |
| | | L-alanyl-L-glutamin | 5 mmol/l |
| | | Murines RANKL | 40 ng/ml |

### Ausführungsbeispiel 3: Auswertung der resorbierten Platten

Der Resorptionsassay beruht auf der Kombination von zwei Methoden miteinander. Dabei werden die resorbierten Platten zuerst mit der von Kossa Färbung (von Kossa J, (1901); modifizert von Barkhatov IM, Roumiantsev SA, Vladimirskaya EB, Afanasyev BV (2008) Composition and functional properties of monolayer cell culture from human umbilical cord blood, Cellular Therapy and Transplantation 1 (2)) gefärbt. Die Methode beruht darauf, dass Calciumionen durch Silberionen ausgetauscht werden, welche nach Reduktion durch Lichteinwirkung als metallisches Silber (schwarz) sichtbar werden. Resorbierte Flächen sind Calcium frei und färben nicht schwarz, während nichtresorbierte Bereiche der Matrix schwarz werden. Gefärbt wurde nach dem Waschen mit Wasser durch Inkubation der Matrix mit Silbernitratlösung (5%) für 60 min. Nach wiederholtem Waschen mit Aqua dest. wurde mit Pyrogallollösung (1%) für 5 bis 10 min entwickelt. Zur Bestimmung der Resorptionsfläche wurden die getrockneten Platten danach mit einem Durchlichtscanner eingescannt und mit Hilfe von z.B. Image Quant ausgewertet. Dabei wurde die eingescannte Fläche festgelegt und weiße von schwarzen Flächen unterschieden. Das Volumen der weißen Fläche kann dann über eine Kalibriergerade (s. Fig. 2) in die tatsächlich resorbierte Fläche umgerechnet werden.

### Vergleichsbeispiel:

Zum Vergleich wurde versucht den aus dem Stand der Technik bekannten OsteoLyse assay von Lonza auf eine andere Osteoklasten-Linie (RAW 264.7) anzuwenden. Dabei wurde nach der Kit-Vorschrift verfahren.

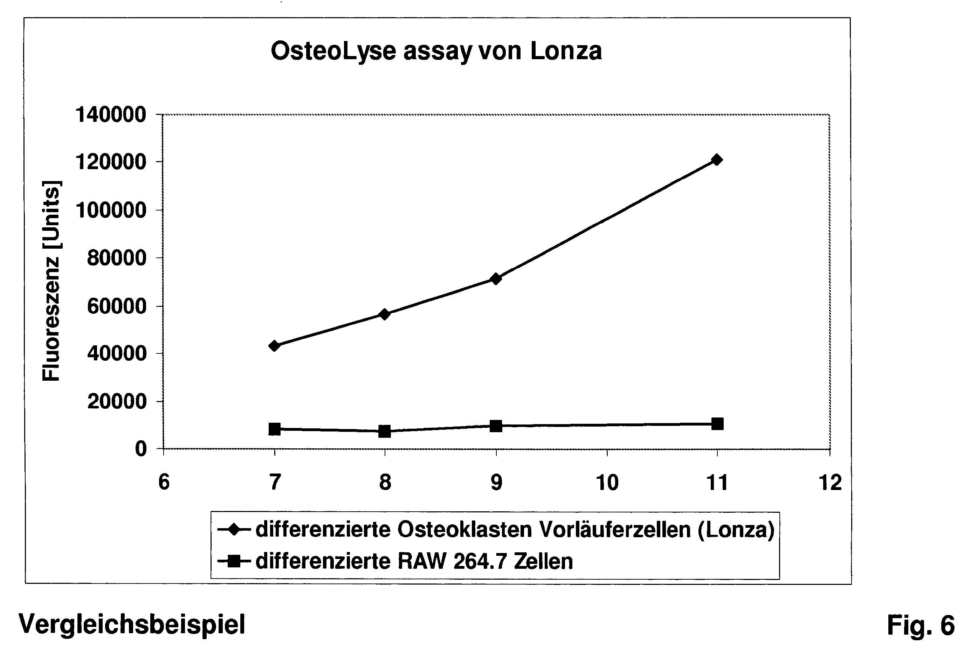

Im Ergebnis zeigt **Fig. 6**, dass der OsteoLyse assay von Lonza mit den RAW 264.7 Zellen nicht funktioniert, während die dem Kit zugehörigen osteoclast precursor cells von Lonza eine zeitabhängige Resorptionsaktivität zeigen.

## Patentansprüche

1. Verfahren zur Bestimmung der Resorptionsaktivität von Osteoklasten mit den Schritten:
a.) Inkubation von Osteoklasten mit einer biomineralisierten, calciumphosphathaltigen Matrix, die durch Osteoblasten *in vitro* erhalten wurde,
b.) Quantifizierung des nicht resorbierten Calciumphosphats.

2. Verfahren nach Anspruch 1, **dadurch gekennzeichnet, dass** die Quantifizierung des nicht resorbierten Calciumphosphats durch Anfärben mit Silbernitrat, vorzugsweise mit der von Kossa Färbung, erfolgt.

3. Verwendung einer Matrix, die durch Abscheidung von Calciumphosphat durch Osteoblasten *in vitro* erhalten wurde, zur Bestimmung der Resorptionsaktivität von Osteoklasten.

4. Verwendung eines Kits enthaltend:
a.) eine Matrix, die durch Abscheidung von Calciumphosphat durch Osteoblasten *in vitro* erhalten wurde,
sowie mindestens einen der weiteren Bestandteile:
b.) Zellkulturmedium,
c.) Fixierungslösung, Waschpuffer,
d.) Sibernitratlösung, Reduktionsmittel,
e.) eine Kalibriergerade,
f.) eine oder mehrere Osteoklastenzellinien,
g.) eine Bedienungsanleitung
zur Bestimmung der Resorptionsaktivität von Osteoklasten.

5. Verwendung des Verfahrens nach Anspruch 1 oder 2 zur Anwendung in der biologischen Forschung, der pharmakologischen Forschung und/oder zur Diagnose der Osteoklastenaktivität und/oder Osteoklastendifferenzierung.

6. Verwendung der in Anspruch 3 definierten Matrix oder des in Anspruch 4 definierten Kits zur Anwendung in der pharmakologischen Forschung und/oder zur Diagnose der Osteoklastenaktivität und/oder Osteoklastendifferenzierung.

## Claims

1. Method for determining the resorption activity of osteoclasts with the steps:
a) incubation of osteoclasts with a bio-mineralized calcium phosphate containing matrix that was obtained by osteoblasts *in vitro*;
b) quantification of the un-resorbed calcium phosphate.

2. Method according to claim 1, **characterized in that** the quantification of the un-resorbed calcium phosphate is realized by staining with silver nitrate, preferably by means of the von Kossa stain.

3. Use of a matrix, obtained by deposition of calcium phosphate by osteoblasts *in vitro,* for determining the resorption activity of osteoclasts.

4. Use of a kit, comprising:
a) a matrix that was obtained by deposition of calcium phosphate by osteoblasts *in vitro,*
as well as at least one of the further components:
b) cell culture medium,
c) fixation solution, washing buffer,
d) silver nitrate solution, reducing agent,
e) a calibration line,
f) one or more osteoclast cell lines,
g) an instruction manual
for determining the resorption activity of osteoclasts.

5. Use of the method according to claim 1 or 2 for use in biological research, pharmacological research and/or for diagnosis of osteoclast activity and/or osteoclast differentiation.

6. Use of the matrix defined in claim 3 or of the kit defined in claim 4 for use in pharmacological research and/or for diagnosis of osteoclast activity and/or osteoclast differentiation.

## Revendications

1. Procédé de détermination de l'activité de résorption d'ostéoclastes comportant les étapes suivantes :
a) incubation des ostéoclastes avec une matrice biominéralisée contenant du phosphate de calcium, qui a été obtenue par des ostéoblastes *in vitro,*
b) quantification du phosphate de calcium non résorbé.

2. Procédé selon la revendication 1, **caractérisé en ce que** la quantification du phosphate de calcium non résorbé se fait par coloration avec du nitrate d'argent, de préférence avec une coloration de Von Kossa.

3. Utilisation d'une matrice, qui a été obtenue par piégeage du phosphate de calcium par les ostéoblastes *in vitro,* pour déterminer l'activité de résorption des ostéoclastes.

4. Utilisation d'un kit contenant :
a) une matrice, qui a été obtenue par piégeage du phosphate de calcium par les ostéoblastes *in vitro,*
ainsi qu'au moins un des autres composants :
b) un milieu de culture cellulaire,
c) une solution de fixation, un tampon de lavage,
d) une solution de nitrate d'argent, un agent de réduction,
e) une droite d'étalonnage,
f) une ou plusieurs lignées cellulaires d'ostéoclastes,
g) un mode d'emploi
afin de déterminer l'activité de résorption des ostéoclastes.

5. Utilisation du procédé selon la revendication 1 ou 2 pour une application en recherche biologique, en recherche pharmacologique et/ou pour le diagnostic de l'activité des ostéoclastes et/ou de la différenciation des ostéoclastes.

6. Utilisation de la matrice définie dans la revendication 3 ou du kit défini dans la revendication 4 pour une application en recherche pharmacologique et/ou pour le diagnostic de l'activité des ostéoclastes et/ou de la différenciation des ostéoclastes.
